# EUROPEAN PATENT APPLICATION

(11) **EP 2 832 369 A1**
(43) Date of publication of application: **04.02.2015**
(21) Application number: 13003810.2
(22) Date of filing: 31.07.2013
(51) Int. Cl.: A61K 45/06, A61K 31/165, A61K 31/19, A61K 31/5375, A61K 31/65, A61K 31/7072, A61P 31/04

(54) **Compositions for ameliorating and/or preventing adverse side effects in antibiotic therapy**

(71) Applicant: Mitogenomix GmbH, 97292 Uettingen (DE)
(72) Inventor: Seibel, Martina, 97292 Uettingen (DE)
(74) Representative: Schüssler, Andrea

(57) **Abstract**

The present invention relates to a pharmaceutical composition comprising: (i) a therapeutically effective amount of at least one antibiotic; and (ii) a therapeutically effective amount of an uridine and/or pyruvate compound. The formulation is prepared in a suitable dosage form for the delivery of antibiotics and the reduction of the known adverse side-effects of the antibiotics is achieved by the added uridine and/or pyruvate compounds when administered to a subject. The present invention neutralizes highly efficient these side-effects without interfering with the antibiotic effect of the drugs. By co-administration of antibiotics and uridine and/or pyruvate compounds, cell's capability to generate energy and synthesize nucleic acids is being supported. Especially side-effects caused by antibiotics that interfere with the cellular and mitochondrial metabolism are effectively removed as secondary actions of the drugs.

Also included are methods of reducing or preventing a failure of treatment for an antibiotic-treatable infection in a patient comprising administering the formulation in a suitable way of administration.

## Description

### Area of the Invention

The present invention relates to a pharmaceutical composition comprising: (i) a therapeutically effective amount of at least one antibiotic; and (ii) a therapeutically effective amount of an uridine and/or pyruvate compound. The formulation is prepared in a suitable dosage form for the delivery of antibiotics and the reduction of the known adverse side-effects of the antibiotics is achieved by the added uridine and/or pyruvate compounds when administered to a subject. The present invention neutralizes highly efficient these side-effects without interfering with the antibiotic effect of the drugs. By co-administration of antibiotics and uridine and/or pyruvate compounds, cell's capability to generate energy and synthesize nucleic acids is being supported. Especially side-effects caused by antibiotics that interfere with the cellular and mitochondrial metabolism are effectively removed as secondary actions of the drugs.

Also included are methods of reducing or preventing a failure of treatment for an antibiotic-treatable infection in a patient comprising administering the formulation in a suitable way of administration.

### Background of the Invention

In 1947, the term "antibiotics" was used to describe agents that are antagonistic to the growth of microorganisms in high dilution (1). Today, the term "antibiotics" has a broader appearance as it encompasses very powerful drugs that fight bacterial infections by killing bacteria or prevent their reproduction. Antibiotics are often identified as low-molecular metabolic substances produced by bacteria, fungi or higher plants. They can be isolated from these sources or are partially or fully synthetic compounds. Substances that are still isolated from living organisms belong to the group of aminoglycosides. Others (e.g. sulfonamides, quinolones or oxazolidinones) are fully synthesized. In general, there are two ways of classifying antimicrobial agents: They can be classified either by the basis of their chemical or biosynthetic origin (natural, seminatural, synthetic) or distinguished based on their biological effect on microorganisms. The latter classification discriminates between bactericidal agents that kill bacteria and bacteriostatic compounds that stall or slow down bacterial growth.

Depending on their way or place of action, antibiotics can be divided in the following subgroups:
- cell wall construction (e.g. β-lactams)
- structure and function of cell membrane (e.g. polymyxin B)
- folic acid synthesis (e.g. sulfonamides)
- protein synthesis (e.g. aminoglycosides)
- structure and function of DNA (e.q. quinolones)
- others (e.g. rifampin)

As all therapeutic drugs to be administered to humans or mammals require extensive testing, new compounds are screened for any negative side-effect before approval for clinical use is given (e.g. FDA). Although most approved antibiotics are considered safe and are often well tolerated by patients, some antibiotics have been associated with a range of adverse effects (2) that can range from fever and nausea to major allergic reactions, including photodermatidis and anaphylaxis. Common side effects include diarrhea and overgrowth of pathogenic bacteria and yeast species. Dramatic side effects, as for instance variations in the hemogram of patients, liver malfunction, reversible and irreversible damage of the bone marrow as well as some forms of leukemia (rare cases) have been reported. As a consequence, compounds with severe side effects experience a far less utilization and are considered as "last-line defense".

Patient's treatment has been further complicated in the last years by the emerging of multidrug resistance microbes (e.g. MRSA, **M**ethicillin **R**esistant **S**taphylococcus **A**ureus). Multidrug resistance is a condition enabling disease causing microorganisms to escape distinct antibiotic, antifungal or antiviral drugs. In this context, even antibiotics previously classified exhibiting severe side-effects have gained major attention again (3) . For example, the phenylpropanoid chloramphenicol is a very effective broad-spectrum antibiotic that functions very well on anaerobic bacteria, gram-positive as well as on gram-negative bacteria. The compound has been used to treat acne, eye infections and for therapy of patients in grave cases of certain meningitis agents, tetracycline-resistant cholera, typhoid, typhus, dysentery, diphtheria, malaria. However, severe side-effects - even leading to death - were reported that degraded this antibiotic compound to the class of "last-line defenders", e.g.:

The *Gray (Grey) Baby Syndrome* is a rare but serious side-effect (4) . It occurs when neonates are treated intravenously with chloramphenicol. Symptoms observed range from an ashen gray color of the skin, vomiting, limp body tone, hypotension, cyanosis, hypothermia to cardiovascular collapse. Due to a yet unknown mechanism, it is thought that a lack of glucuronidation reactions occurring in the baby leads to an accumulation of toxic metabolites (5).

Chloramphenicol can also induce a damage of bone marrow tissue that in turn can lead to severe forms of anemia (either reversible or aplastic anemia). As a consequence thereof, a malfunctioning erythropoiesis is frequently seen (6). In very rare cases, a coincidence of chloramphenicol treatment and leukemia in childhood has been described (7).

The side-effects of antibiotics are frequently caused by interference with cell's metabolism or protein synthesis in mammalia. The latter is particular important to mitochondria as the ribosomes in these organelles are evolutionary closely linked to bacterial ribosomes. Consequently, a drug directed to interfere with bacterial protein synthesis will impede mitochondrial protein synthesis and thus will lead to dramatic effects on the overall metabolism. Mitochondria have been identified to house a variety of essential metabolic pathways. Among those, the generation of energy through the mitochondrial oxidative phosphorylation system plays a pivotal role to sustain cell's energy demand. However, to keep up the energy requirements under drugs that intercept the protein synthesis, cell's metabolism will be shifted towards the far less effective glycolytic pathway, thereby producing lactate. Thus, energy synthesis becomes the rate limiting step and is involved in the experienced side-effects of the antibiotics (e.g. chloramphenicol, linezolid, tigezycline etc.). Additionally, the diminishing number of OXPHOS complexes stall the uridine biosynthesis pathway so that the cell cycle of fast dividing cells can be arrested.

### Object

The object of the present invention is to provide compositions for ameliorating and/or preventing adverse side effects in antibiotic therapy.

### Brief Description of the Present Invention

This object is solved by the subject-matter of present independent claim 1. Preferred embodiments are mentioned in the dependent claims.

The invention provides a pharmaceutical composition comprising:
(i) a therapeutically effective amount of at least one antibiotic; and
(ii) a therapeutically effective amount of an uridine and/or pyruvate compound.

The inventors have found out that side-effects of antibiotics that interfere with mitochochondrial protein synthesis cause a reduction of the energy generating system (enzymes of the oxidative phosphorylation system, OXPHOS). Fast dividing cells are mostly affected, as they cannot keep the number of OXPHOS complexes constant in the daughter cells. Thus, it is known from other studies that enzymes of OXPHOS are diluted as they cannot be re-synthesized under drug influence. Within 4-5 days, the number of OXPHOS complexes is under the level needed to sustain cells energy demand (approximately 8-12 %) so that a biochemical pathway shift to glycolysis will occur. In contrast, cells in resting state are less affected as they display turnover rates (half-life) of mitochondrially synthesized proteins of 8-20 days. Thus, they can cope with the blocked biosynthesis of OXPHOS so that even after a 30 days treatment, symptoms cannot be detected within this time span.

Hence, side effects developing in patients treated with antibiotic drugs are caused by two distinct ways:
- Firstly, due to the inhibition of mitochondrial protein synthesis, these antibiotics shift the energy generating pathway towards glycolysis. Thus, glycolysis needs to cover almost the entire energy demand of cells and needs to function at its best.
- Secondly, due to the diminished rate of OXPHOS complexes, a second problem, distinct from the energy underrun, kicks in: electrons from the dihydroorotate dehydrogenase (DHODH) are fed exclusively into the OXPHOS via the mitochondrial ubiquinol/ubiquinone cycle. If OXPHOS enzymes become rate limiting, the DHODH pathway comes to a halt as electrons cannot be released from the DHODH-redox reaction. The enzyme (DHODH) is linked to the essential uridine biosynthesis pathway. Thus, shortening in cell's uridine supply causes consequently a stalling of the cell cycle.

When metabolism is shifted towards anaerobic glycolysis under antibiotic treatment, cells produce lactate from pyruvate that is released into the blood. As a consequence of the occurring metabolic switch, the NAD⁺/NADH ratio is shifted towards NADH, because it cannot be re-oxidized by the few remaining OXPHOS complexes. This situation leads to a shortening of NAD⁺ and pyruvate within the cell so that essential biochemical pathways are blocked. When pyruvate is administered, both problems are solved:
- Firstly, the inner cellular pyruvate pool increases so that stalled biochemical reactions can be restarted again
- Secondly, as the NADH level can be reduced by production of lactate from the administered pyruvate, reactions previously stalled by the high NADH level can be restarted again.

Cell's metabolism is additionally challenged by the diminished uridine pool that is caused by the stalled DHODH reaction. It is known that cells uridine pool is filled by three distinct pathways:
- Firstly, the cell is fed from the salvage pathway. That is: all uridines (pyrimidines) utilized within the cell (e.g. the building blocks all nucleic acids or UDP release from UDP-glucuronic acid) are reused upon breakdown. The pyrimidine level resulting from the recycling is sufficient to sustain cell's demand in a resting cell.
- Secondly, the pyrimidine hunger of dividing cells can be saturated from the freshly synthesized building blocks via the DHODH pathway.
- Thirdly, pyrimidines can be taken up by the cell. Thus extracellular sources can additionally bring in the required building blocks for cells in mitosis.

When uridine (pyrimidine) is administered in addition to the antibiotic, cell's uridine pool is refilled. Thus, biochemical reactions diminished by the antibiotic side-effect requiring UDP (e. g. glucoronidation or nucleic acid synthesis) are bolstered up so that reactions are restarted or accelerated to a normal speed. Thus, administration of one or both of pyruvate and/or uridine ameliorates and/or prevents all symptoms seen in antibiotic treated patients (e.g. gray baby syndrome; deteriorated blood building system due to energy and/or mitotic arrest of blood cells; failure of glucuronidation reaction).

### Brief Description of the Figures:

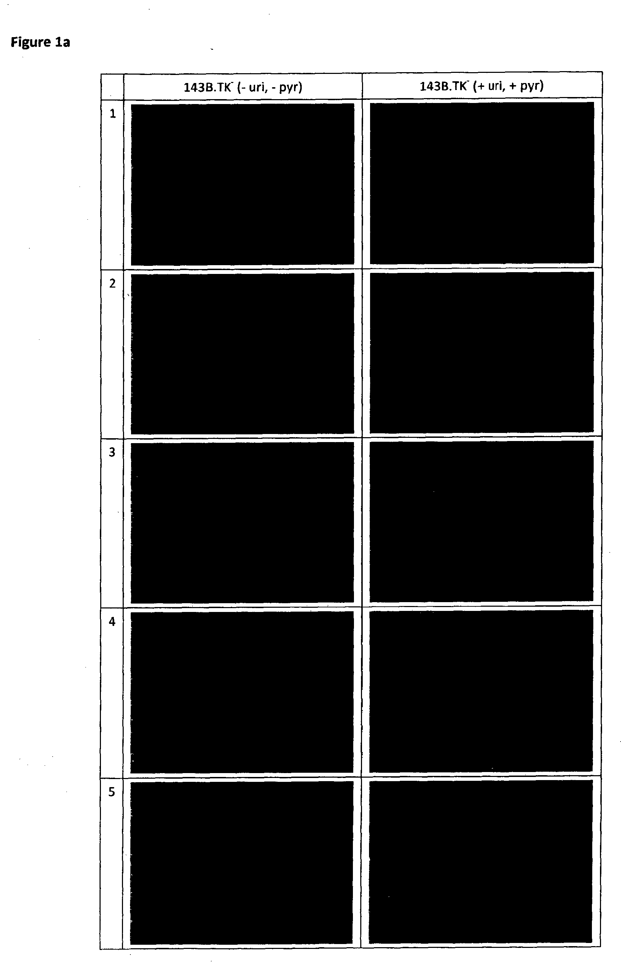

**Figure 1a****: *Side effect of chloramphenicol (or linezolid or tigezycline) on dividing human cells.***
   The panel displays cell growth of a green colorized human osteosarcoma cell line (143B.TK⁻) by fluorescence microscopy. ***Left panel:*** 143B.TK⁻ cells containing a green colorizing gene (GFP, green color) were seeded and grown to the original density in normal growth medium (left, 1). At day 1, medium was changed to allow cell growth under pyruvate and uridine free conditions; the medium was supplemented with chloramphenicol (or linezolid, or tigezycline; see example 1). At day 4 (image 2), cell growths retarded and comes to a halt. At day 6 (image 3), the imaged colony starts to disperse and diminishes. At day 8 (image 4), cell number further declines and cells ceased to exist (day 10). ***Right panel:*** 143B.TK⁻ cells containing a green colorizing gene (GFP, green color) were seeded and grown to the original density in normal growth medium (right, 1). At day 1, medium was changed to allow cell growth under controlled pyruvate and uridine supply; the medium was supplemented with chloramphenicol (or linezolid, or tigezycline; see example 1). It turned out, that growth behavior of the cells was not altered by the antibiotic as the colony expanded regularly to day 10.

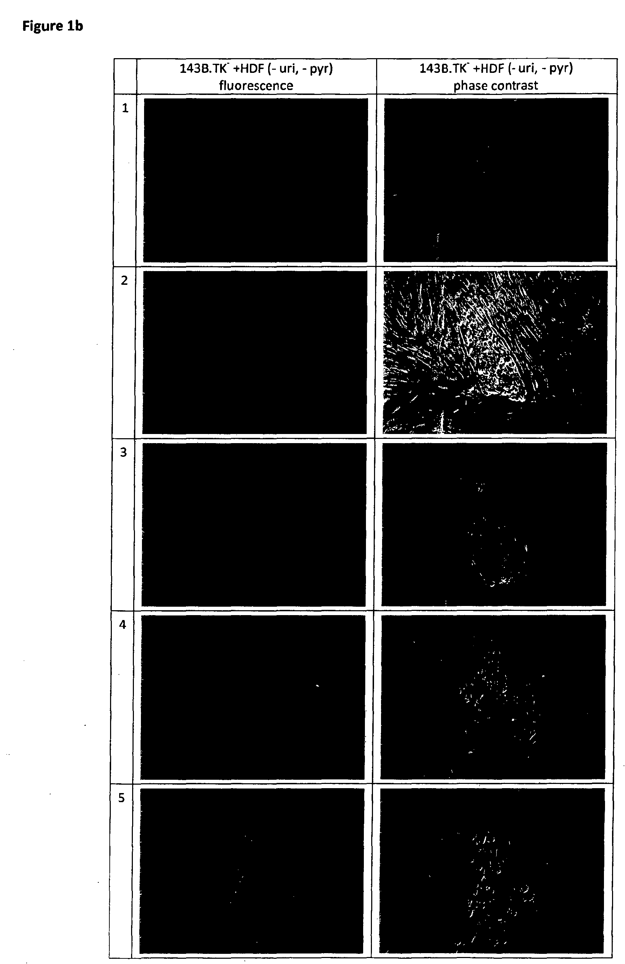

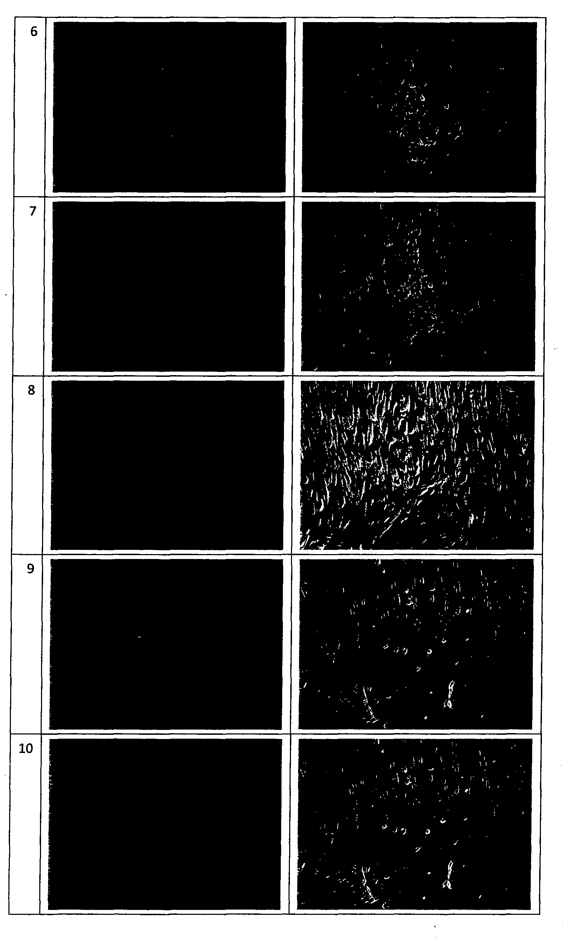

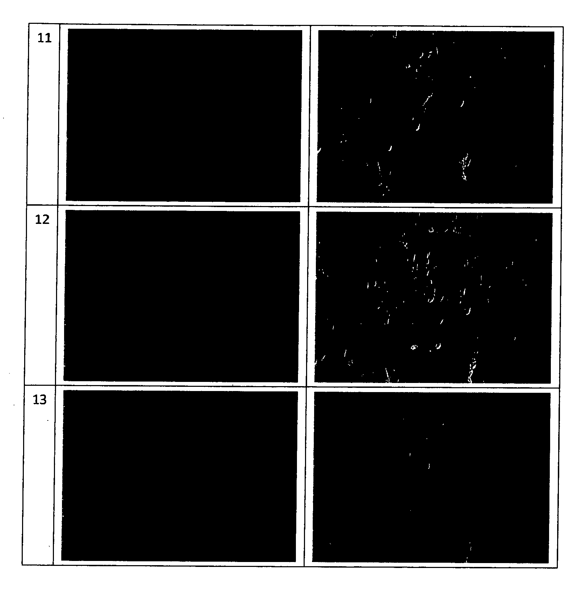

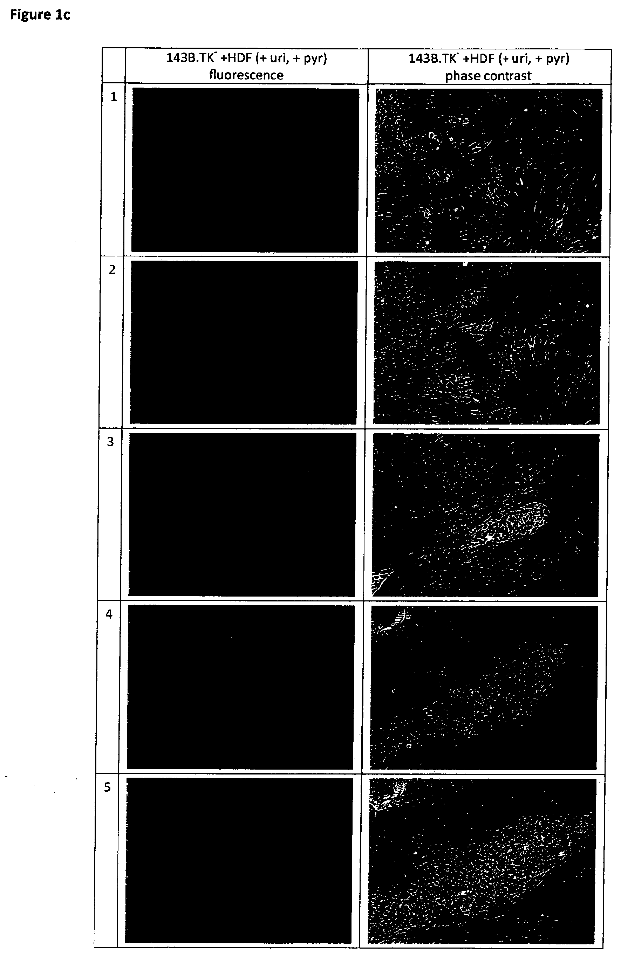

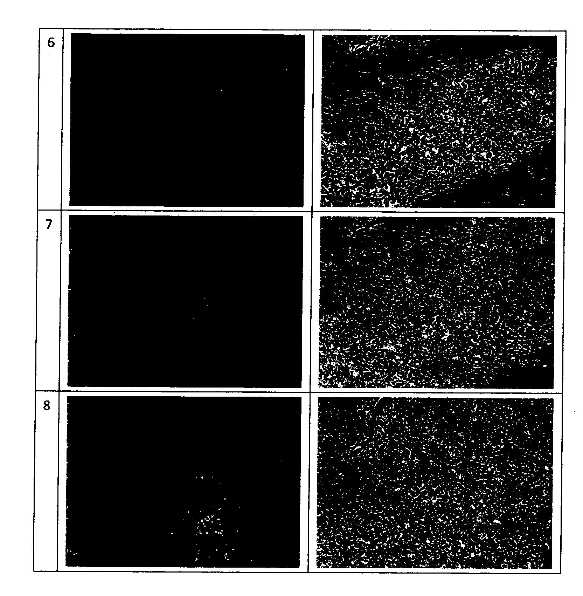

**Figure 1b** **and** **1c****: *Influence of antibiotic on cell cycle in the absence or presence of pyruvate and uridine.***
   The panels display co-cultures grown on petri-dishes that contain a human dermal fibroblast (HDF) monolayer inoculated with GFP-colorized 143B.TK⁻ cells imaged by fluorescence microscopy and phase contrast microscopy. The HDF cells represent a resting post-mitotic tissue, while the GFP-colorized 143B.TK⁻ cells mimic mitotic active cells.

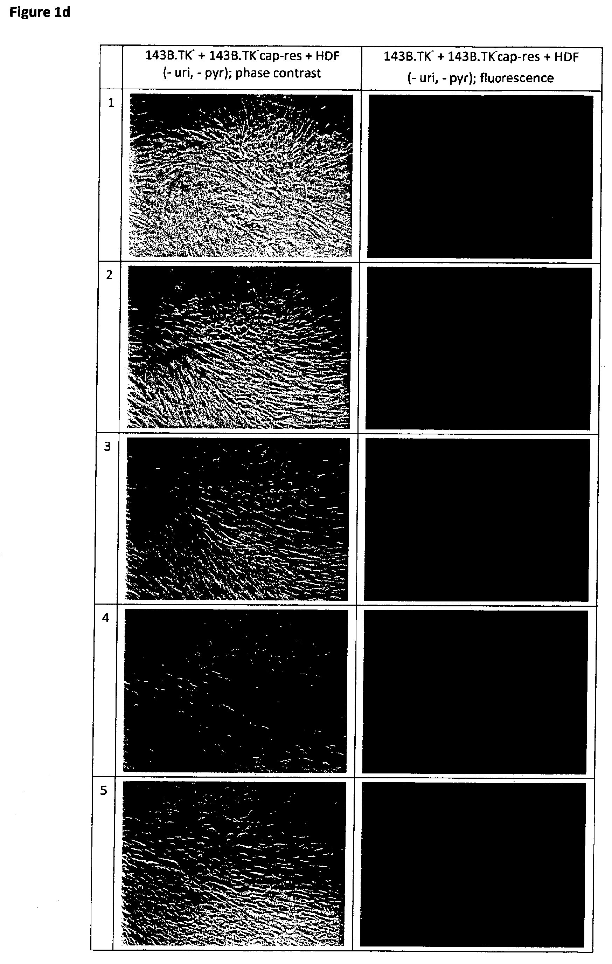

**Figure 1b****, left panel:** Without pyruvate and uridine, the side effect of chloramphenicol (or linezolid, or tigezycline) can be visualized on co-cultures similarly to the effect seen in figure 1. Approximately at day 4 of culture, the mitotic active colony enlarges and experiences growth retardation. At day 6 dispersion of the colony starts and finally ceases to exist at day 13. **Right panel:** Same area as seen in the left panel. There is no change in cell vitality seen on the HDF monolayer. Thus, the side-effect is primarily seen in mitotic active cells.
**Figure 1c****, left panel:** Co-cultured cells in the presence of pyruvate and uridine do not display any side-effect induced by chloramphenicol (or linezolid or tigezycline). The mitotic cells grow completely regularly. At day 8, the entire area is overgrown by the GFP-colorized 143B.TK⁻ cells. **Right panel:** Phase-contrast images of the same area as seen in the left panel. There is no change in cell vitality seen on the HDF monolayer mimicking post-mitotic or resting cells for more than 30 days.
**Figure 1d****: *Co-cultures of human dermal fibroblasts and GFP-colorized 143B.TK⁻and with dsRED-colorized 143B.TK⁻CAP-res cells in the presence* of *chloramphenicol and absence of uridine and pyruvate.***
   The panels display co-cultures grown on petri-dishes that contain a human dermal fibroblast (HDF) monolayer inoculated with GFP-colorized 143B.TK⁻ cells and dsRED-colorized 143B.TK⁻CAP-res cells. The dsRED-colorized 143B.TK⁻CAP-res cells contain a mitochondrial genotype that renders the cells chloramphenicol resistant. Images were taken by phase contrast (left panel) and fluorescence microscopy (right panel).
   **Left panel:** Phase contrast image of same area as seen in the right panel. There is no change in cell vitality seen on the HDF monolayer mimicking post-mitotic or resting cells.
   **Right panel:** Colonies of approximately similar sizes were chosen (green and red). As can be seen in the images taken on subsequent days, the mitotic active cells (green color code) diminishes upon chloramphenicol side-effects when uridine and pyruvate are not supplied, whereas the chloramphenicol resistant cell line is not influenced by the side-effect of chloramphenicol and grew regularly.

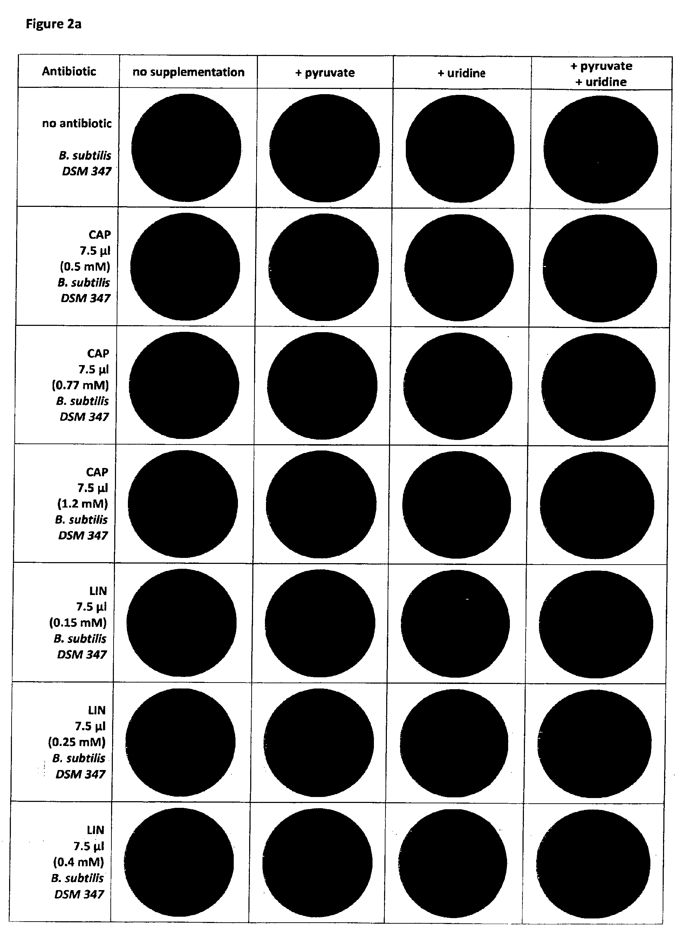

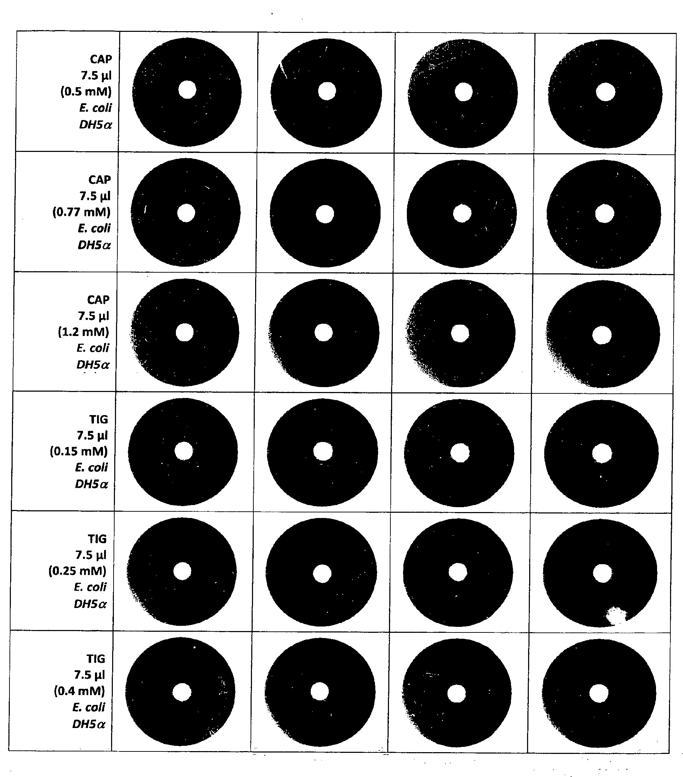

**Figure 2a****: Antibiotic effect is not altered by co-administration of pyruvate, uridine or pyruvate + urine.**
   The panel displays the non-growth zone of bacteria (*B. subtilis* DSM 347 or *E. coli* DH5α) that is caused by antibiotic diffusion from the soaked paper-pads into the growth plate. The size of the non-growth zone is a measure for antibiotic activity.

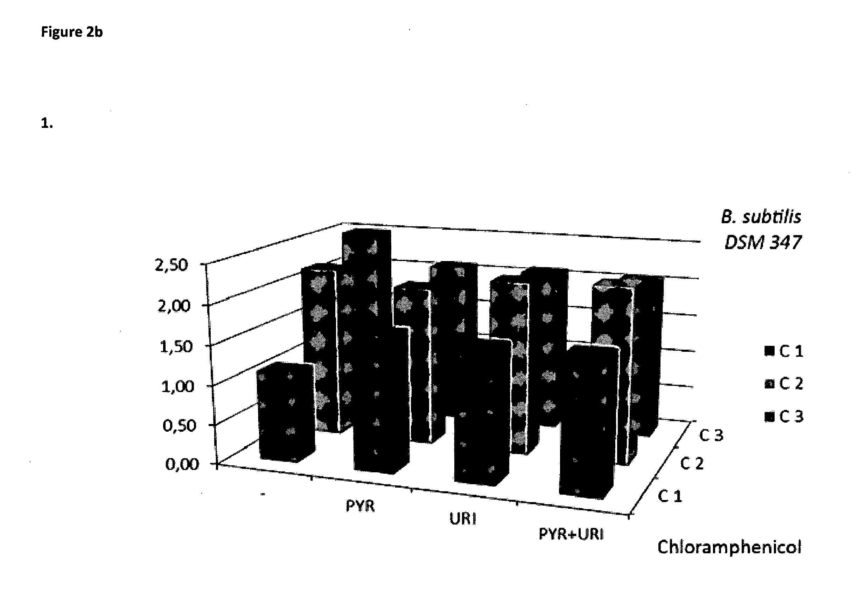

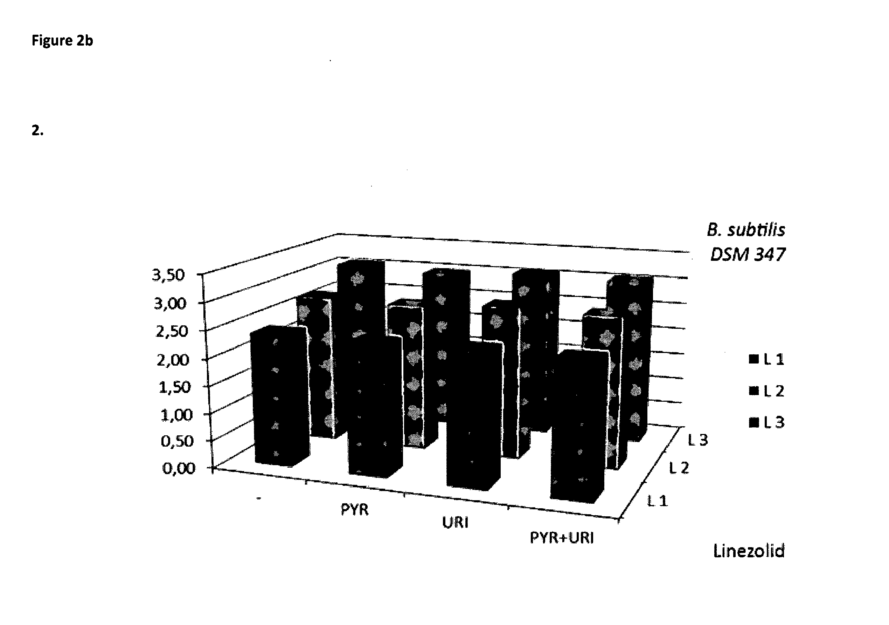

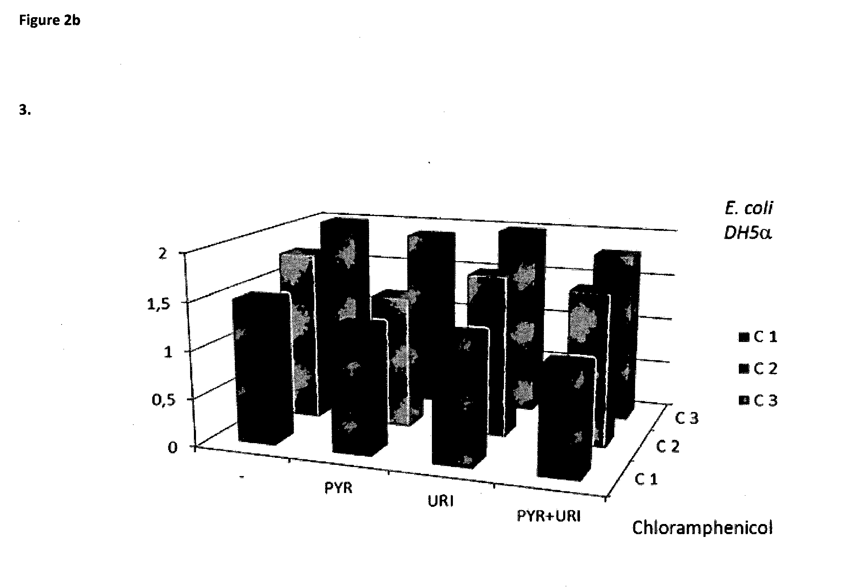

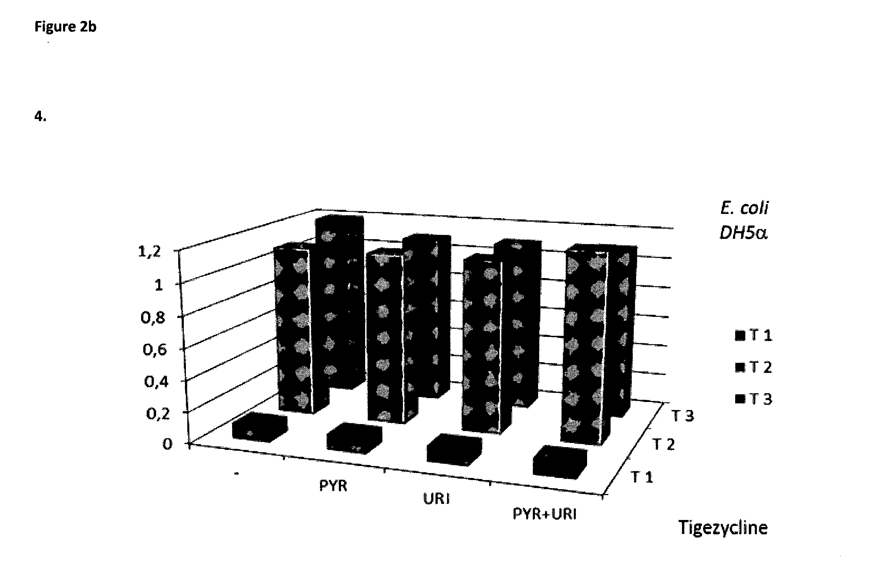

**Figure 2b****: Quantitative analysis of the unaltered antibiotic effect during co-administration of pyruvate, uridine or pyruvate + uridine.**
   **First panel:** Antibiotic effect of chloramphenicol (C1: 0.5 mM CAP, C2: 0.77 mM CAP, C3: 1.2 mM CAP) on *B. subtilis* DSM 347. Antibiotic effect increases with concentration of antibiotic, but is independent of pyruvate, uridine or pyruvate + uridine supplementation.
   **Second panel:** Antibiotic effect of linezolid (L1: 0.15 mM LIN, L2: 0.25 mM LIN, L3: 0.4 mM LIN) on *B. subtilis* DSM 347. Antibiotic effect increases with concentration of antibiotic, but is independent of pyruvate, uridine or pyruvate + uridine supplementation.
   **Third panel:** Antibiotic effect of chloramphenicol (C1: 0.5 mM CAP, C2: 0.77 mM CAP, C3: 1.2 mM CAP) on *E*. *coli* DH5α. Antibiotic effect increases with concentration of antibiotic, but is independent of pyruvate, uridine or pyruvate + uridine supplementation.
   **Fourth panel:** Antibiotic effect of tigezycline (T1: 0.15 mM TIG, T2: 0.25 mM TIG, T3: 0.4 mM TIG) on *E. coli* DH5α. Antibiotic effect increases with concentration of antibiotic, but is independent of pyruvate, uridine or pyruvate + uridine supplementation.

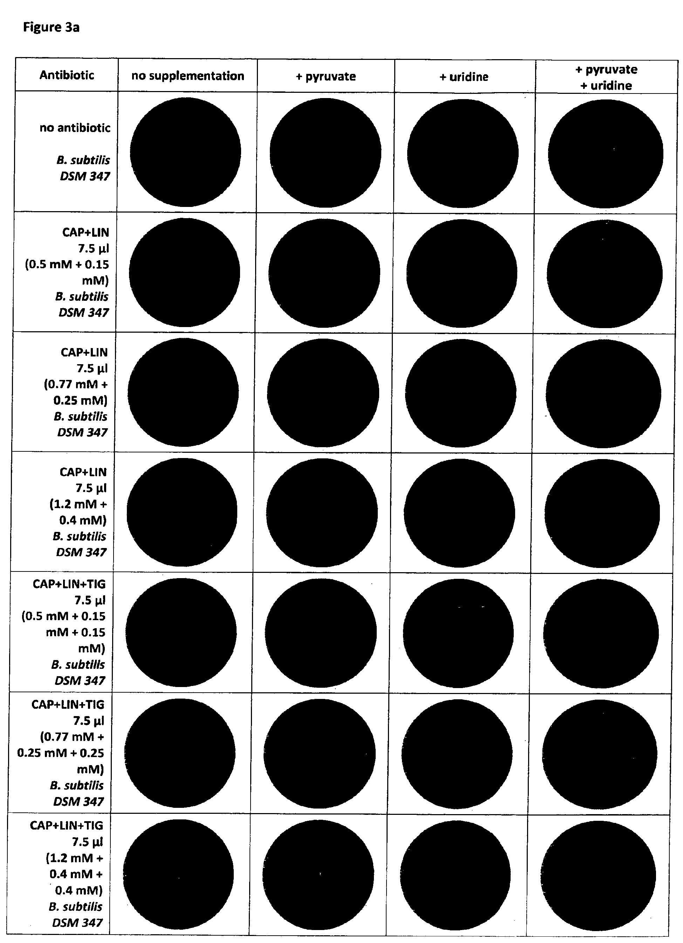

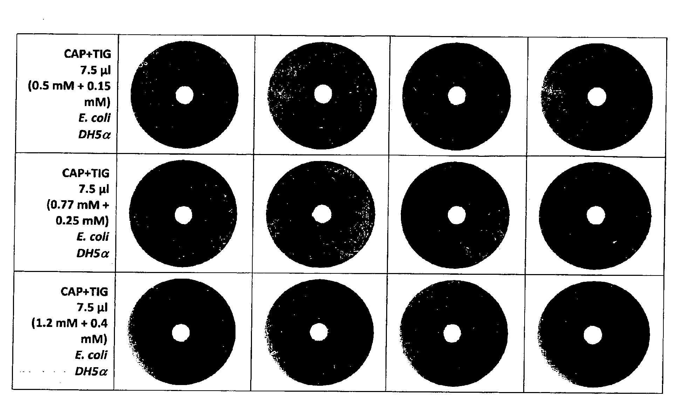

**Figure 3a****: Antibiotic effect is not altered by combination of antibiotics and co-administration of pyruvate, uridine or pyruvate + urine.**
   The panel displays the non-growth zone of bacteria (*B. subtilis* DSM 347 or *E. coli* DH5α) that is caused by antibiotic diffusion from the soaked paper-pads into the growth plate. The size of the non-growth zone is a measure for antibiotic activity. The following antibiotic combinations were used: CAP + LIN, CAP + LIN + TIG, CAP + TIG.

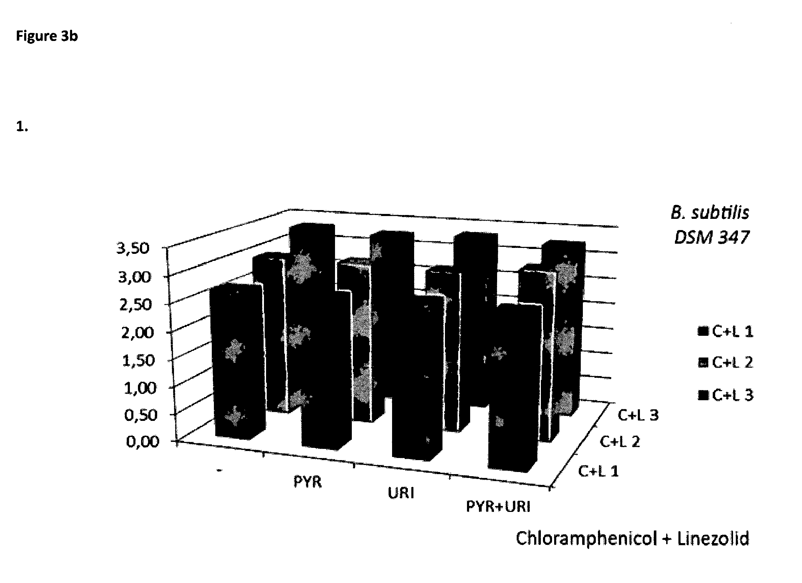

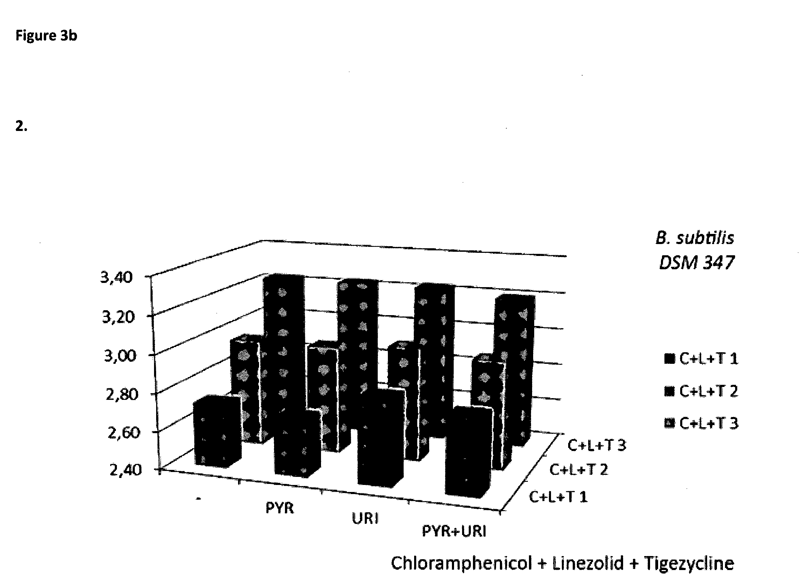

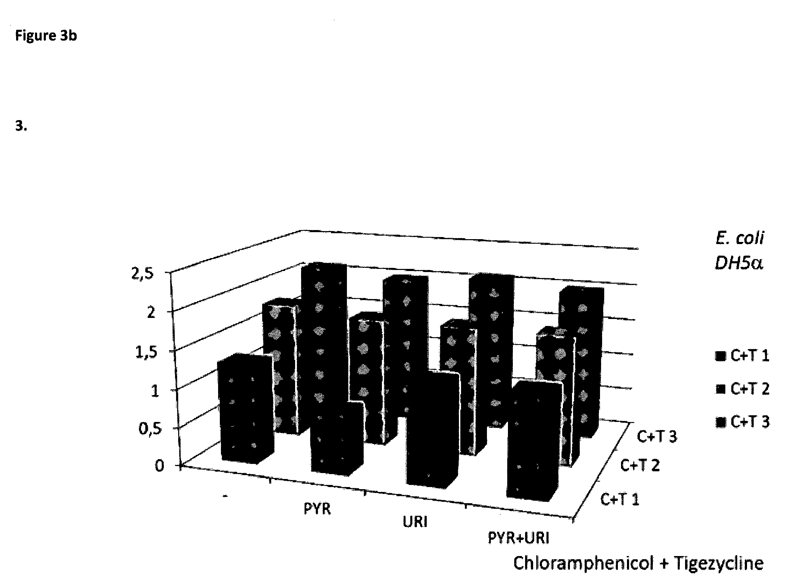

**Figure 3b****: Quantitative analysis of the antibiotic combination effect (CAP+LIN; CAP+LIN+TIG; CAP+TIG) during co-administration of pyruvate, uridine or pyruvate + uridine.**
   **First panel:** Antibiotic effect of chloramphenicol + linezolid (C + L 1: 0.5 mM CAP + 0.15 mM LIN, C + L 2: 0.77 mM CAP + 0.25 mM LIN, C + L 3: 1.2 mM CAP + 0.4 mM LIN) on *B. subtilis* DSM 347. Antibiotic effect increases with concentration of antibiotic, but is independent of pyruvate, uridine or pyruvate + uridine supplementation.
   **Second panel:** Antibiotic effect of chloramphenicol + linezolid + tigezycline (C + L +T 1: 0.5 mM CAP + 0.15 mM LIN + 0.15 mM TIG, C + L + T 2: 0.77 mM CAP + 0.25 mM LIN + 0.25 mM TIG, C + L + T 3: 1.2 mM CAP + 0.4 mM LIN + 0.4 mM TIG) on *B. subtilis* DSM 347. Antibiotic effect increases with concentration of antibiotic, but is independent of pyruvate, uridine or pyruvate + uridine supplementation.
   **Third panel:** Antibiotic effect of chloramphenicol + tigezycline (C + T 1: 0.5 mM CAP + 0.15 mM TIG, C + T 2: 0.77 mM CAP + 0.25 mM TIG, C + T 3: 1.2 mM CAP + 0.4 mM TIG) on *E. coli* DH5α. Antibiotic effect increases with concentration of antibiotic, but is independent of pyruvate, uridine or pyruvate + uridine supplementation.

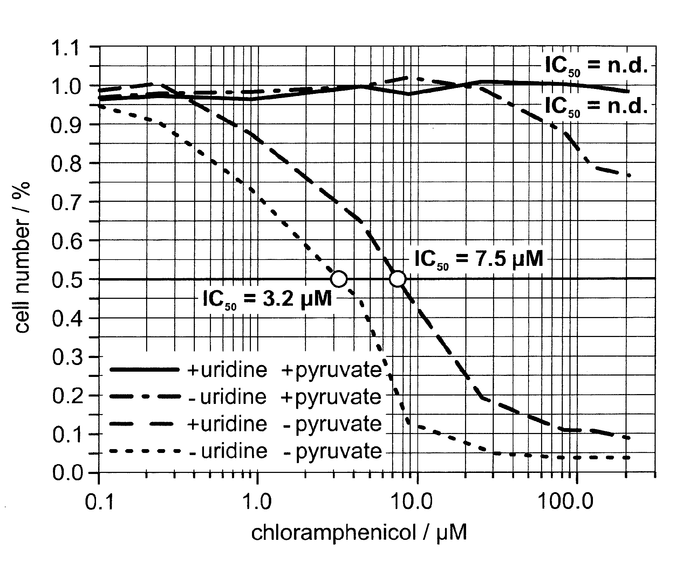

**Figure 4****: *Determination of IC₅₀ values of chloramphenicol in the presence or absence of pyruvate and*/*or uridine.***
   Vitality of 143B.TK⁻ cells was tested using resazurin (see example 4). The image displays a plot of cell number/% vs the chloramphenicol concentration /µM. It can be easily seen, that cells without uridine and pyruvate supplementation experienced a severe toxicity. IC₅₀ could be determined at a concentration of approximately 3.2 µM chloramphenicol. Cultures complemented with uridine experienced a shift of IC₅₀ towards approximately 7.5 µM chloramphenicol, indicating a stabilization of cell vitality. When complementation was carried out with pyruvate or pyruvate and uridine, both IC₅₀ values could not be determined, because they were beyond scale (IC₅₀ >300 µM chloramphenicol). Thus, in the plotted concentration range, uridine and pyruvate supplementation neutralized completely the side effects that are caused by chloramphenicol treatment without additives. Cell's vitality was indistinguishable from cell growth without chloramphenicol treatment.

### Abbreviations:

CAP: Chloramphenicol
LIN: Linezolid
TIG: Tigezycline

### Detailed Description of the Present Invention

The invention provides a pharmaceutical composition comprising:
(i) a therapeutically effective amount of at least one antibiotic; and
(ii) a therapeutically effective amount of an uridine and/or pyruvate compound.

In the present invention the terms "formulation" and "(pharmaceutical) composition" are used interchangeably.

"Subject" as used herein refers to humans and non-human primates (e.g. guerilla, macaque, marmoset), livestock animals (e.g. sheep, cow, horse, donkey, pig), companion animals (e.g. dog, cat), laboratory test animals (e.g. mouse, rabbit, rat, guinea pig, hamster), and any other organism who can be benefit from the agents of the present disclosure. There is no limitation on the type of animal that could benefit from the presently described agents. A subject regardless of whether it is a human or non-human organism may be referred to also as a patient, individual, animal, host, or recipient.

The term "about" as used herein, means in quantitative terms plus or minus 5%, or in another embodiment plus or minus 10%, or in another embodiment plus or minus 15%, or in another embodiment plus or minus 20%.

It has been found out by the inventors that it is possible to ameliorate and/or eliminate the reported side effects of antibiotics if the administration of antibiotics is accompanied by the supplementation with an uridine and/or pyruvate compound.

The term "an uridine and/or pyruvate compound" means at least one of an uridine or a pyruvate compound, or a mixture of both of them. It also encompasses two or more of each of them or mixtures or two or more of each.

The most common side effects of antibiotic therapy to be diminished and/or eliminated are: rash, diarrhea, abdominal pain, nausea/vomiting, drug fever, hypersensitivity (allergic) reactions, serum sickness, vaginal candidiasis, renal (kidney) toxicity, ototoxicity (hearing loss), dizziness, nystagmus, headache, liver toxicity, eosinophilia (elevated white blood cells), anorexia, hemolytic anemia, peripheral neuropathy, dizziness, reddish-orange body fluids, flushing, hypotension, itching, phlebitis, taste, taste alterations, photosensitivity , tooth discoloration in children, lethargy, insomnia, photosensitivity , pseudomembranous colitis, jaundice, metallic taste.

Unexpectedly, it has been found that the combination of uridine and/or pyruvate compounds and one or more antibiotics results in no significant diminishment of the effectiveness of the antibiotic(s), or alteration of the pharmacokinetics of the antibiotic.

According to the present invention any antibiotic may be used. Preferentially, those antibiotics can be selected that belong to the group of protein synthesis inhibitors or those presenting with side-effects related to the activity of cellular protein synthesis. Suitable antibiotics include those of the classes aminoglycosides, ansamycins, carbacephmes, cephalosporins, glycopeptides, macrolides, penicillins, polypeptides, quinolones, sulfonamides, tetracyclines, glycycyclines, oxazolidinones, amphenicols, pleuromutilins, lincosamides, streptogramins, steroid antibacterials and mixtures thereof.

Specific antibiotics that may be used (alone or in combination) include, for example, amikacin, gentamicin, kanamycin, neomycin, netilmicin, streptomycin, tobramycin, paromomycin, geldanamycin, herbimycin, loracarbef, ertapenem, doripenem, imipenem/cilastatin, meropenem, cefadroxil, duricef, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftobiprole, teicoplanin, vancomycin, azithromycin, clarithromycin, dirithromycin, erythromycin, roxithromycin, troleandomycin, telithromycin, spectinomycin, aztreonam, amoxicillin, ampicillin, azlocillin, carbenicillin, cloxacillin, dicloxacillin, flucloxacillin, mezlocillinm, meticillin, nafcillin, oxacillin, penicillin, piperacillin, ticarcillin, ciprofloxacin, enoxacin, gatifloxacin, levofloxacin, lomefloxacin, fodaximicin, moxifloxacin, norfloxacin, ofloxacin, trovafloxacin, grepafloxacin, sparfloxacin, temafloxacin, mafenide, sulfonamidochrysoidine sulfacetamide, sulfadiazine, ulfamethizole, sulfanilamide, sulfasalizine, sulfisoxazole, trimethoprim, trimethroprim-sulfamethoxazole (co-trimoxazole) (TMP-SMX), doxycycline, minocycline, oxytetracycline, tetracycline, chloramphenicol, tigezycline, linezolid , clindamycin, lincomycin, ethambutol, myambutol, fosfomycin, fusidic acid, furazolidone, isoniazid, linezolid, metronidazole, mupirocin, nitrofurantoin, platensimycin, pyrazinamide, quinupristin/dalfopristin, rifampicin, thiamphenicol, tinidazole, dapsone, clofazimine dihydrostreptomycin, framycetin, ribostamycin, arbekacin, bekanamycin, dibekacin tobramycin, hygromycin B, sisomicin, isepamicin, verdamicin, astromicin, chlortetracycline clomocycline, demeclocycline, lymecycline, meclocycline, metacycline, penimepicycline, rolitetracycline, torezolid, eperezolid, posizolid, radezolid, azidamfenicol, florfenicol, retapamulin, tiamulin, valnemulin, flurithromycin, josamycin, midecamycin, miocamycin, oleandomycin, rokitamycin, spiramycin, tylosin, ketolides, cethromycin, solthromycin, pirlimycin, pristinamycin, virginiamycin and mixtures thereof.

In an embodiment, the formulation may include a combination of 2, of 3, of 4, of 5 or of 6 or more antibiotics or agents. The combination or single antibiotic(s) chosen will necessarily depend on the nature and type of infection that is to be treated in each circumstance and the medical particulars of the individual patient. Such selection is well within the scope of a person of skill in the art. In preferred embodiments of the present invention at least one of the antibiotics is selected from the subgroup of protein synthesis inhibitors or these antibiotics that display side-effects involved in protein synthesis.

In another embodiment of the present invention at least one of the antibiotics is selected from the subgroup of chloramphenicol, linezolid or tigezycline.

Antibiotic-treatable infections may include, for example, bacterial sinusitis, various sexually transmitted diseases of bacterial origin, pharyngitis, otitis, especially otitis media, bacterial bronchitis, bacterial pneumonia, diverticulitis, urinary tract invections, skin and skin structure infections, cellulitis, abscesses, furuncles, impetigo, pyoderma, wound infections, acne, nail infections, chronic bacterial prostatitis, acute pyelonephritis, and/or infections caused by any one or more of *Haemophilus influenza, Haemophilus parainfluenzae, Moraxella catarrhalis, Staphylococcus aureus, Staphylococcus epidermidis, Staphylococcos saprophyticus, Pseudomonas aeruginosa, Serratia marcescens, Escherichia coli, Klebsiella pneumonia, Streptococcus pneumonia, Streptococcus neumoniaeparainfluenzae, Streptococcus pyogenes, Chlamydia pneumonia, Legionella pneumophila, Mycoplasma pneumonia, Vibrio cholera, Bacillus anthracis, Eikenella corrodens, Neisseria gonorrhoeae, Enterococcus faecalis, Enterobacter* cloacae, *Proteus mirabilis,* bacteria of the genus *Bacteroides,* including *Bacteroides fragilis,* bacteria of the genus *Fusobacterium,* and bacteria of the genus *Peptostreptococcus,* and MRSA infections.

The dosage of the composition according to the invention, in particular of the antibiotic component, is determined by the physician on the basis of the patient-specific parameters, such as age, weight, sex, severity of the disease, *etc*. The mode of treatment and the dosage regimen of each the antibiotic(s) and the uridine and/or pyruvate material(s) will be mainly dictated by the specific antibiotic(s). In general, the unit dose and the dosage regimen will parallel that of the antibiotic if it were to be administered alone. For example, if the antibiotic recommended dosage is 200 mgs at two hour intervals, the antibiotic dosage in the formulation of the present invention will remain substantially the same, as would the frequency of administration of the entire formulation. However, the therapeutically effective dosage of any specific antibiotic or agent will vary somewhat from patient to patient, and will depend upon the condition of the patient and the dosage form.

The terms "dosage regimen" or "mode of treatment" refer to a timely sequential or simultaneous administration of the components (i) and (ii) of the composition of the present invention. This means that components (i) and (ii) may be provided in a unit dosage form with physical contact to each other (e.g. one single tablet or solution) or as separate entities (e.g. two tablets or solutions) to be taken simultaneously or with a certain time difference. This time difference may be between 0.5 hour and 1 day, preferably between 1 hour and 5 hours. In one embodiment, the (i) antibiotic(s) and (ii) uridine and/or pyruvate are physically segregated within the dosage form, for example, separated by an acrylic or cellulose membrane in a tablet or capsule. In an alternative embodiment, both ingredients (i) and (ii) are in physical contact within the dosage form, such as for example, in a mixed powder or granules form (free or formed into a tablet or capsule) or a liquid. The mode of administration of (i) and (ii) is preferably simultaneously, i.e. at substantially the same time, from separate entities. In a preferred embodiment, the antibiotic is administered via another administration way than the uridine and/or pyruvate. In this regard it may be advantageous to administer either the antibiotic or the uridine and/or pyruvate intravenously and the other orally. If uridine and pyruvate are administered as component (ii) they may be taken together or with a certain time difference. They may be provided in a single unit dosage form (e.g. one single tablet or solution) or as separate entities (e.g. two tablets or solutions) to be taken simultaneously or with a certain time difference. This time difference may be same as mentioned before.

In the present invention the uridine compound is administered to increase a level of uridine in a tissue, plasma, or cell of a subject.

In the present invention the term "uridine" or "uridine compound" means uridine, uridine phosphate(s), uridine precursors, uridine derivatives, uridine metabolites, uridine sources, uracil, pyrimidines and/or uridine-based compounds. In other words, the term "uridine (compound)" as used herein refers to any uridine phosphate, uridine precursor, uridine metabolite, uridine-based, uracil-based or pyrimidine-based compound, or salt thereof mentioned above. In another embodiment, "uridine" referes to any uridine or related compound that is known in the art.

In one embodiment, the uridine compound that is administered in the present invention is a uridine-5'monophosphate (UMP). In another embodiment, the uridine is a uridine-5'-diphosphate (UDP). In another embodiment, the uridine is a uridine-5'triphosphate (UTP). In another embodiment, the uridine is UDP-glucose. Each possibility represents a separate embodiment of the present invention.

In another embodiment, the uridine compound is an uridine precursor. In one embodiment, the uridine precursor that is administered is a cytidine-5'monophosphate. In another embodiment, the uridine precursor that is administered is a cytidine-5'-diphosphate (CDP). In another embodiment, the uridine precursor that is administered is a CDP-glucose. In another embodiment the uridine precursor is e.g. orotic acid or orotidylate. In another embodiment, the uridine precursor that is administered is any pharmacologically acceptable uridine derivative or metabolite known in the art.

In another embodiment, a uridine derivative is administered. The term "derivative" in one embodiment refers to a compound chemically related to uridine in such a way that uridine is converted to the derivative in a subject's body. In another embodiment, "derivative" refers to a compound chemically related to uridine in such a way that the derivative is converted to uridine in a subject's body. In one embodiment, the conversion occurs via one or more stable intermediates. In another embodiment, the conversion occurs directly. Each possibility represents a separate embodiment of the present invention.

In another embodiment, a uridine metabolite is administered in methods of the present invention.

In other embodiments, uridine-based compounds other than uridine itself serve as uridine sources or uridine precursors. These are, in some embodiments, uridine-rich products like algae, salts of uridine like uridine phosphates, acylated uridine or the like. In another embodiment, therapeutically or pharmacologically effective doses of acyl derivatives of uridine or mixtures thereof, e.g. those disclosed in U.S. Pat. No. 5,470,838, are administered.

In another embodiment, the uridine source is cytidine-diphosphocholine (CDP-choline; citicholine).

In another embodiment, a salt of the uridine precursor, derivative or source is utilized in a method of the present invention. In one embodiment, the salt is UMP disodium. In another embodiment, the salt is any other pharmacologically acceptable salt of a uridine precursor or derivative. In another embodiment, the composition that is administered comprises the salt of the uridine or precursor or derivative thereof as the sole active ingredient.

In another embodiment, a mixture of two or more of the above mentioned uridine compounds is administered. Each type of uridine precursor, derivative, metabolite, or source represents a separate embodiment of the present invention.

In one embodiment, the uridine compound is administered in a dosage of between about 20 milligrams (mg) and 50 grams (g) per day. In another embodiment, the uridine compound is administered in a dosage of about 50 mg-30 g per day. In another embodiment, the dosage is about 75 mg-20 g; 100 mg-15 g; 100 mg-10 g; 200 mg-8 g; 400 mg-6 g; 600 mg-4 g; 800 mg-3 g; 1-2.5 g; 1.5-2 g, about 2 g per day.

According to the present invention the pyruvate compound is pyruvic acid, a salt thereof or methyl or ethyl pyruvate or pyruvate derivatives as for example creatine pyruvate. Additionally, all compounds that can be easily converted within the cell to pyruvate (e. g. phosphoenolpyruvate, 2-phosphoglycerate, 3-phosphoglycerate, 1,3-biphosphoglycerate, glyceraldehyde 3-phosphate, dihydroxyacetone phosphate, fructose-1,6 biphosphate, fructose 6-phosphate, glucose 6-phosphate, glucose) can be utilized. In a preferred embodiment the pyruvate compound is sodium salt of pyruvate.

In one embodiment, the pyruvate compound is administered in a dosage of between about 100 milligrams (mg) and 10 grams (g) per day. In another embodiment, the pyruvate compound is administered in a dosage of about 200 mg-9 g per day. In another embodiment, the dosage is about 500 mg-8 g; 750 mg-7 g; 1-6.5g, 1.5-6 g, 2-5 g, or 2.5-3.5 g per day.

The pharmaceutical composition of the present invention may be prepared and administered in any dosage form suitable for administration to the subject's body. It is formulated to be compatible with its intended route of administration. Examples of routes of administration include parenteral (e.g. intravenous, intradermal, subcutaneous), oral, by inhalation, systemic (e.g. topical, transmucosal), and rectal administration.

In a preferred embodiment, the formulation may be prepared as a tablet, liquid, gel, a suspension, an emulsion or a solution.

Solutions or suspensions used for parenteral, intradermal, or subcutaneous application can include the following components: a sterile diluents such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates, or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes, or multiple dose vials made of glass or plastic.

Pharmaceutical compositions suitable for an injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor® EL (BASF, Parsippany, N.J.), or phosphase buffered saline (PBS).

In all cases, the composition must be sterile and should be fluid to the extent that easy syringability exists. It should be stable under the conditions of manufacture and storage and must be preserved against the contaminating action of microorganism such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganism can be achieved by various antibacterial and antifungal agents, for example, parabens, chlorobutanol, phenol, ascorbic acid, thimerosal, and the like. According to embodiments, isotonic agents, for example, sugars, polyalcohols such as manitol, sorbitol, or sodium chloride in the composition are added. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preparation is prepared by vacuum drying or freeze-drying, which yields a powder of the active ingredient plus any additional desired ingredient from a previously sterile-filtered solution thereof.

In a further preferred embodiment the formulation of the present invention is suitable for oral administration. Oral compositions generally include an inert diluents or an edible carrier. For the purpose of oral therapeutic administration, the active compound can be incorporated with excipients and used in the form of tablets, troches, chewie, gel caps, soft gel or capsules, e.g. gelatin capsules. Oral compositions can also be prepared using a fluid carrier for use as a mouthwash. Pharmaceutically compatible binding agents, or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid. Primogel, or corn starch; a lubricant such as magnesium searate or sterotes; a glidant such as colloidal silicon dioxide; a sweetening agent such as sucrose or saccharin; or a flavoring agent such as peppermint, methyl salicylate, or strawberry, cherry, grape, lemon, or orange flavoring.

For administration by inhalation, the compounds are delivered in the form of an aerosol spray from pressured container or dispenser which contains a suitable propellant, e.g., a gas such as carbon dioxide, or a nebulizer.

According to embodiments, intravitreal injection is accomplished using PLGA-based microparticles or nanoparticules (liposomes). PEG-based formulas may also be used. Accordingly, the other methods for injectable pharmaceutical compositions are expressly contemplated for intravitreal injection.

Systemic administration can also be transmucosal or transdermal. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds can also be prepared in the form of suppositories (e.g., with conventional suppository bases such as cocoa butter and other glycerides) or retention enemas for rectal delivery.

In addition to the other forms of delivery, the compounds are deliverable via eye drop or intraocular injection. With respect to eye drops, the compositions of the present disclosure optionally comprise one or more excipients intended for topical application to the eye or nose. Excipients commonly used in pharmaceutical compositions intended for topical application to the eyes, such as solutions or sprays, include, but are not limited to, tonicity agents, preservatives, chelating agents, buffering agents, surfactants and antioxidants. Suitable tonicity-adjusting agents include mannitol, sodium chloride, glycerin, sorbitol and the like. Suitable preservatives include p-hydroxybenzoic acid ester, benzalkonium chloride, benzododecinium bromide, polyquaternium-1 and the like. Suitable chelating agents include sodium edentate and the like. Suitable buffering agents include phosphates, borates, citrates, acetates and the like. Suitable surfactants include ionic and nonionic surfactants, though nonionic surfactants are preferred, such as polysorbates, polyethoxylated castor oil derivatives and oxyethylated tertiary octylphenol formaldehyde polymer (tyloxapol). Suitable antioxidants include sulfites, ascorbates, BHA and BHT.

The formulation of the present invention may include any additional component as desired, as long as such components do not substantially erode the effectiveness of the antibiotic or the uridine and/or pyruvate compounds. Such components may include, for example, thickeners, sweeteners, flavorants, fragrances, additional pharmaceuticals, glycine, mannitol, silicone dioxide, silica gels, binders, vitamins, carriers (such as, for example, glycerin, starches, celluloses, chitins, starches, water, alcohol) and minerals.

In the following particular preferred embodiment of the present invention are described. The life threating situation of a mitotic cell treated with an antibiotic can be rescued, when pyruvate and/or uridine are administered simultaneously with the antibiotic. As described in detail in example 1 (Figure 1a-d), the adverse side effect of antibiotics can be ameliorated and/or prevented, when the antibiotic is co-administered with pyruvate and/or uridine. Example 1 shows also that in co-cultures of mitotic and resting cells (Figure 1b, 1c) the resting cells are not challenged by the antibiotic, because they do contain still enough OXPHOS complexes so that biochemical pathways are not altered. That the mitochondrial protein synthesis is the central point of antibiotic side effects is demonstrated by Figure 1d: Cells harboring an artificial mitochondrial CAP resistance mutation (red cells) are not influenced by CAP as they grow also in uridine and pyruvate deficient media. In contrary, the green cells (normal, cap sensitive cells) deteriorate when treated with CAP without uridine and pyruvate supplementation.

That the antibiotic effect is not affected by the co-administration of pyruvate and/or uridine is demonstrated by example 2: The antibiotic effect was tested in dependence of pyruvate, uridine or pyruvate + uridine complementation and was solely depending on the applied antibiotic dosage. Moreover, also a combination of antibiotics as exemplified in example 3 did not show any influence of the antibiotic capacity when supplementation with pyruvate, uridine or pyruvate + uridine occurred.

To prove whether or not the antibiotic effect is attenuated or diminished when pyruvate, uridine or pyruvate + uridine are co-administered, *B. subtilis* and *E. coli* strains were tested in the presence and absence of the supplementing compounds. It turned out (see example 2, Figure 2a and 2b) that the antibiotic effect is independent of co-administration of pyruvate, uridine or pyruvate + uridine. Moreover, even combinations of antibiotics in the presence of pyruvate, uridine or pyruvate + uridine did not alter the antibiotic effect of the drugs (see example 3, Figure 3a and 3b).

To determine the actual impact of antibiotic + supplementation by pyruvate, uridine or pyruvate + uridine onto cells performance, vitality tests were carried out utilizing resazurin (see example 4, Figure 4). This test resulted in a shift of IC₅₀ side-effect-values towards a far better tolerance of the drug. The IC₅₀ side-effect-values of chloramphenicol for example shifted from 3.2 µM of cells without supplementation to 7.5 µM of cells supplemented with uridine. Complementation with pyruvate or pyruvate + uridine yielded shifts that were beyond our scale (> 300 µM) and were practically indistinguishable from cell viability when cells were not treated with an antibiotic. Thus, administering antibiotics together with pyruvate, uridine or pyruvate + uridine leads to a complete loss of ameliorating or adverse side effects.

The following examples show preferred embodiments of the present invention but are not intended to limit the present invention in any aspect.

### Example 1:

### Side-effect of antibiotics and its neutralization

In order to understand the side effects of antibiotics, we incubated cultured human cells with antibiotics that are known to impede cell's metabolism. The experiments were carried out with chloramphenicol, tigezycline or linezolid under otherwise identical conditions. In details, 143B.TK⁻ cells were plated on petri-dishes and cultured in DMEM F0435 (w/o pyruvate), supplemented with 5% dialyzed fetal calf serum. Cells were treated with chloramphenicol (150 µg/ml culture medium, 450 µM; or linezolid, or tigezycline) and medium was exchanged on a daily basis. After 4 days of regular cell growth in the presence of the antibiotic, cell growth retarded and came to a halt after 6 days. After additional 4 days, cells ceased to exist (see figure 1a).

To understand the influence of the antibiotic on cell cycles state, a co-culture was established that was based on a primary fibroblasts monolayer (mimicking cells at resting state) that was inoculated with 143B.TK⁻ cells (mimicking cells in mitotic state, dividing cells). Under otherwise identical conditions, the co-cultures were subjected to the treatment with antibiotics (150 µg/ml culture medium, 450 µM; or linezolid, or tigezycline) in DMEM F0435 (w/o pyruvate), supplemented with 5% dialyzed fetal calf serum.

As seen in the above described experiment (Figure 1a), cell growth of the 143B.TK-cells came to a halt after 4-5 days and ceased to exist after additional 6 days (see figure 1b and 1c). However, the fibroblast layer was completely unaffected and vital to start regrowth again (proven by executing scratches through the monolayer; "wound-healing" set in immediately). When the same experiment was carried out in the presence of uridine and/or pyruvate under otherwise identical conditions, the cell growth of 143B.TK⁻ cells was completely unaffected, demonstrating that the observed side effect relies exclusively on energetic deficiency and/or cell cycle stalling. Both side-effects could be removed by co-administering uridine and/or pyruvate or derivatives thereof.

To see whether or not the side-effect of these antibiotics reside within the mitochondria of the cell, a co-culture was established that was made of a primary fibroblast monolayer inoculated with 143B.TK⁻ and 143.TK⁻CAP^{res} cells. The latter cell line is identical to the 143B.TK- cell line, except that these cells carry a CAP resistant mutation within the 16S rRNA gene located within the mitochondrial genome and are differently color coded. Upon chloramphenicol administration, the 143B.TK- cell line exhibited the same behavior as described above: cells stopped growth after 4-5 days and ceased to exist after additional 6 days whereas CAP-resistant cells exhibited no limitation in cell growth (see figure 1d). When these co-cultures were supplemented with pyruvate and/or uridine (pyruvate: 100 µg/ml sodium pyruvate; uridine: 50 µg/ml uridine; supplementation of growth medium) both 143B.TK- cell lines were indistinguishable in their growth rate, demonstrating that the severe side-effect of the antibiotic was completely abolished.

### Example 2:

### Antibiotic effect is independent of co-administration of uridine and/or pyruvate

To prove whether or not the antibiotic effect of the drugs is rendered by the co-administration of uridine and/or sodium pyruvate, *E. coli* strain DH5α or *B. subtilis* strain DSM 347) were plated on LB-agar plates or APT-plates that were used as is or supplemented with uridine (50 µg/ml), sodium pyruvate (1 mM) or uridine (50 µg/ml) + pyruvate (1 mM).

Paper test pads (Rotiolabo, 6mm, Roth, Karlsruhe) were soaked with 7.5 µl of antibiotic-solution (concentrations used: 0.5 mM CAP; 0.77 mM CAP; 1.2 mM CAP; 0.15 mM LIN; 0.25 mM LIN; 0,4 mM LIN; 0.15 mM TIG; 0.25 mM TIG; 0.4 mM TIG) and placed on top of the agar plates (CAP- and LIN-solutions on APT/*B*. *subtilis* plates, TIG-solutions on LB/*E*. *coli* plates). Petri dishes were incubated over night at 37°C and analyzed by measuring the size of the non-growth zone of bacteria surrounding the antibiotics soaked paper pads (see figure 3a, and 3b).

All dilutions of antibiotics applied presented an antibiotic effect independent from the co-administered substances (uridine and/or pyruvate). As background control, similar experiments were carried out without antibiotic solution under otherwise identical conditions. As expected, when pyruvate or uridine was administered solely or in combination *without* antibiotic, non-growths zones were obsolete. Thus, antibiotic effects of chloramphenicol, tigezycline or linezolid are solely based on the antibiotic compound and are not rendered by the co-administered compounds uridine and/or pyruvate.

### Example 3

### Antibiotic effect seen by combination of antibiotic compounds in the presence of uridine, pyruvate or uridine + pyruvate

To prove whether or not the antibiotic effect of the drugs is rendered by combining antibiotics and co-administering uridine and/or sodium pyruvate, *B. subtilis* strain DSM 347 was plated on APT-plates (*E. coli* strain DH5α were grown on LB plates) that were used as is or supplemented with uridine (50 µg/ml), sodium pyruvate (1 mM) or uridine (50 µg/ml) + pyruvate (1 mM).

Paper test pads (Rotiolabo, 6mm, Roth, Karlsruhe) were soaked with 7.5 µl of antibiotic-solution (concentrations used: 0.5 mM CAP; 0.77 mM CAP; 1.2 mM CAP; 0.15 mM LIN; 0.25 mM LIN, 0.4 mM LIN; 0.15 mM TIG, 0.25 mM TIG; 0.4 mM TIG; 0.5 mM CAP + 0.15 mM LIN; 0.77 mM CAP + 0.25 mM LIN; 1.2 mM CAP + 0.4 mM LIN; 0.5 mM CAP + 0.15 mM LIN + 0.15 mM TIG; 0.77 mM CAP + 0.25 mM LIN + 0.25 mM TIG; 1.2 mM CAP + 0.4 mM LIN + 0.4 mM TIG; 0.5 mM CAP + 0.15 mM TIG; 0.77 mM CAP + 0.25 mM TIG; 1.2 mM CAP + 0.4 mM TIG) and placed on top of the agar plates (CAP- and LIN-solutions on APT/*B*. *subtilis* plates, TIG-solutions on LB/*E*. *coli* plates). Petri dishes were incubated over night at 37°C and analyzed by measuring the size of the non-growth zone of bacteria surrounding the antibiotics soaked paper pads (see figure 2a, and 2b).

All dilutions of antibiotics applied presented an antibiotic effect independent from the co-administered substances (uridine and/or pyruvate). As background control, similar experiments were carried out without antibiotic solution under otherwise identical conditions. As expected, when pyruvate or uridine was administered solely or in combination *without* antibiotic, non-growths zones were obsolete. Thus, antibiotic effects of chloramphenicol, tigezycline or linezolid are solely based on the antibiotic compound and are not rendered by the co-administered compounds uridine and/or pyruvate.

### Example 4

### Determination of IC₅₀ values of antibiotics in the presence of uridine and/or pyruvate

The half maximal inhibitory concentration (IC₅₀) is a measure of the effectiveness of a compound in inhibiting biological or biochemical function. This quantitative measure indicates, how much of a particular drug or compound is needed to inhibit a given biological process by half. In this case, the IC₅₀ value was determined for the side-effect of the antibiotic on human cells in the presence or absence of pyruvate and/or uridine. IC₅₀ values were determined utilizing resazurin (Cell-Quanti-Blue Cell Viability Assay Kit, Biotrend Chemikalien GmbH, Köln), a fluorescent dye indicating vital living or dead cells. For the experiment, 24-well culture dishes were seeded with a dilution of 143B.TK⁻ cells (10³ cells each). The culture medium was composed of DMEM FG0445 (containing sodium pyruvate, high-glucose, glutamine; at the discretion of the supplier) supplemented with 5% FCS and 0.5 % uridine. After an initial growth of two days, cells were washed in PBS. From here, cells were grown in DMEM F0435 (containg high-glucose, without pyruvate, without glutamine; at the discretion of the supplier) supplemented with 2 % glutamine and 5% FCS. Depending on the subsequent experiments, cell growth medium was further supplemented with:
1. w/o chloramphenicol, w/o pyruvate, w/o uridine
2. w chloramphenicol (50 nM - 300 µM), w/o pyruvate, w/o uridine
3. w chloramphenicol (50 nM - 300 µM), w/o pyruvate, w uridine (50 µg/ml)
4. w chloramphenicol (50 nM - 300 µM), w pyruvate (100 µg/ml), w/o uridine
5. w chloramphenicol (50 nM - 300 µM), w pyruvate (100 µg/ml), w uridine (50 µg/ml)

Growth medium was changed every 12 hours. The experiment was carried out for 10 days.

For analysis displayed in Figure 4, cell vitality (cell number) was measured utilizing resazurin. Measures of sets 2-5 were normalized by measure 1. Thus, when cell vitality under chloramphenicol treatment is similar to normal cell growth (measure 1), the ratio of (measure 2, 3, 4, 5) /(measure 1) expressed in % should stay close to 1. As can be seen in Figure 4, IC₅₀ of CAP treatment without uridine or pyruvate supplementation was approximately 3.2 µM. When cells were supplemented with uridine under otherwise identical conditions, the IC₅₀ of CAP shifted to 7.5 µM. When pyruvate or pyruvate + uridine were added to the growth medium, IC₅₀ of CAP was out of range (> 300 µM).

It can be easily seen from the displayed data that full supplementation (pyruvate + uridine) leads to an indistinguishable vitality of cells grown in CAP (ratio - 1) when compared to cells grown in CAP without supplementation.

Having described preferred embodiments of the invention, it is to be understood that the invention is not limited to the precise embodiments, and that various changes and modification may be effected therein by those skilled in the art without departing from the scope or spirit of the invention as defined in the appended claims.

### Reference List

(1) Waksman, S. A. "What is an antibiotic or an antibiotic substance?" Mycologia. 39.5 (1947): 565-69.
(2) Slama, T. G., et al. "A clinician's guide to the appropriate and accurate use of antibiotics: the Council for Appropriate and Rational Antibiotic Therapy (CARAT) criteria." Am.J.Med. 118 Suppl 7A (2005): 1S-6S.
(3) Falagas, M. E., A. P. Grammatikos, and A. Michalopoulos. "Potential of old-generation antibiotics to address current need for new antibiotics." Expert.Rev.Anti.Infect.Ther. 6.5 (2008): 593-600.
(4) McIntyre, J. and I. Choonara. "Drug toxicity in the neonate." Biol.Neonate 86.4 (2004): 218-21.
(5) Brunton, L. L. Chapter 46. Protein Synthesis Inhibitors and Miscellaneous Antibacterial Agents. Goodman & Gilman's The Pharmacological Basis of Therapeutics (11th ed.). 2006. McGraw-Hill.
(6) Yunis, A. A. "Chloramphenicol toxicity: 25 years of research." Am.J.Med. 87.3N (1989): 44N-8N.
(7) Shu, X. O., et al. "Chloramphenicol use and childhood leukaemia in Shanghai." Lancet 2.8565 (1987): 934-37.

## Claims

1. A pharmaceutical composition comprising:
(i) a therapeutically effective amount of at least one antibiotic; and
(ii) a therapeutically effective amount of an uridine and/or pyruvate compound.

2. The pharmaceutical composition of claim 1, wherein the antibiotic is selected from the antibiotic classes of aminoglycosides, ansamycins, carbacephmes, cephalosporins, glycopeptides, macrolides, penicillins, polypeptides, quinolones, sulfonamides, tetracyclines, glycycyclines, oxazolidinones, amphenicols, pleuromutilins, lincosamides, streptogramins, steroid antibacterials and mixtures thereof.

3. The pharmaceutical composition of claim 2 wherein the antibiotic is selected from the group consisting of amikacin, gentamicin, kanamycin, neomycin, netilmicin, streptomycin, tobramycin, paromomycin, geldanamycin, herbimycin, loracarbef, ertapenem, doripenem, imipenem/cilastatin, meropenem, cefadroxil, duricef, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftobiprole, teicoplanin, vancomycin, azithromycin, clarithromycin, dirithromycin, erythromycin, roxithromycin, troleandomycin, telithromycin, spectinomycin, aztreonam, amoxicillin, ampicillin, azlocillin, carbenicillin, cloxacillin, dicloxacillin, flucloxacillin, mezlocillinm, meticillin, nafcillin, oxacillin, penicillin, piperacillin, ticarcillin, ciprofloxacin, enoxacin, gatifloxacin, levofloxacin, lomefloxacin, fodaximicin, moxifloxacin, norfloxacin, ofloxacin, trovafloxacin, grepafloxacin, sparfloxacin, temafloxacin, mafenide, sulfonamidochrysoidine sulfacetamide, sulfadiazine, ulfamethizole, sulfanilamide, sulfasalizine, sulfisoxazole, trimethoprim, trimethroprim-sulfamethoxazole (co-trimoxazole) (TMP-SMX), doxycycline, minocycline, oxytetracycline, tetracycline, chloramphenicol, tigezycline, linezolid , clindamycin, lincomycin, ethambutol, myambutol, fosfomycin, fusidic acid, furazolidone, isoniazid, linezolid, metronidazole, mupirocin, nitrofurantoin, platensimycin, pyrazinamide, quinupristin/dalfopristin, rifampicin, thiamphenicol, tinidazole, dapsone, clofazimine dihydrostreptomycin, framycetin, ribostamycin, arbekacin, bekanamycin, dibekacin tobramycin, hygromycin B, sisomicin, isepamicin, verdamicin, astromicin, chlortetracycline clomocycline, demeclocycline, lymecycline, meclocycline, metacycline, penimepicycline, rolitetracycline, torezolid, eperezolid, posizolid, radezolid, azidamfenicol, florfenicol, retapamulin, tiamulin, valnemulin, flurithromycin, josamycin, midecamycin, miocamycin, oleandomycin, rokitamycin, spiramycin, tylosin, ketolides, cethromycin, solthromycin, pirlimycin, pristinamycin, virginiamycin and mixtures thereof.

4. The pharmaceutical composition of claim 3 wherein the antibiotic is chloramphenicol, linezolid or tigezycline.

5. The pharmaceutical composition of any of claims 1 to 4, wherein the uridine is selected from the group consisting of uridine, uridine phosphate(s), uridine precursors, uridine derivatives, uridine metabolites, uridine sources, uracil, pyrimidines and/or uridine-based compounds.

6. The pharmaceutical composition of any of claims 1 to 3, wherein the pyruvate is sodium salt of pyruvate.

7. The pharmaceutical composition of any of claims 1 to 4, wherein (i) the antibiotic and (ii) the uridine and/or pyruvate are in a unit dosage form to be administered simultaneously.

8. The pharmaceutical composition of any of claims 1 to 4, wherein (i) the antibiotic and (ii) the urdine and/or pyruvate are provided as separate entities.

9. The pharmaceutical composition of any of claims 1 to 6 for use in preventing or minimizing adverse side effects in antibiotic therapy.

10. The pharmaceutical composition of any of claims 1 to 6 for the use of claim 9, wherein the adverse side effects in antibiotic therapy to be prevented or minimized are rash, diarrhea, abdominal pain, nausea/vomiting, drug fever, hypersensitivity (allergic) reactions, serum sickness, vaginal candidiasis, renal (kidney) toxicity, ototoxicity (hearing loss), dizziness, nystagmus, headache, liver toxicity, eosinophilia (elevated white blood cells), anorexia, hemolytic anemia, peripheral neuropathy, dizziness, reddish-orange body fluids, flushing, hypotension, itching, phlebitis, taste, taste alterations, photosensitivity , tooth discoloration in children, lethargy, insomnia, photosensitivity, pseudomembranous colitis, jaundice and/or metallic taste.
